# EUROPEAN PATENT SPECIFICATION

(11) **EP 0 749 739 B1**
(45) Date of publication and mention of the grant of the patent: **02.11.2000**
(21) Application number: 95830254.9
(22) Date of filing: 19.06.1995
(51) Int. Cl.: A61F 13/15

(54) **Perforated dual topsheets for absorbent articles**
Zweifache perforierte Decklagen für absorbierende Artikel
Couche supérieure double avec perforations pour article absorbant

(43) Date of publication of application: 27.12.1996
(73) Proprietor: THE PROCTER & GAMBLE COMPANY, Cincinnati, Ohio 45202 (US)
(72) Inventor: Divo, Michael, D-61381 Friedrichsdorf (DE); Coles, Peter, I-66023 Francavilla al Mare, Chieti (IT); Fornasari, Giancarlo, Pescara (IT)
(74) Representative: Hirsch, Uwe Thomas

(56) References cited:
- EP-A- 0 165 807
- EP-A- 0 203 821
- EP-A- 0 205 286
- EP-A- 0 545 423
- US-A- 4 609 518

## Description

### Field of the invention

The present invention relates to absorbent articles particularly sanitary napkins having layered topsheets. In particular a first layer receiving the liquids to be absorbed comprises apertures of at least 1.4 mm² area in a film. These large apertures provide exceptionally good liquid intake performance for the absorbent article. A second film layer provides liquid transfer to an absorbent structure through apertures of less than 1.4 mm² area. Additionally the second layer provides visual masking of absorbent liquids in the area of the large apertures of the first topsheet layer.

### Background of the invention

Sanitary articles such as sanitary napkins, baby diapers, absorbent inserts, and absorbent adult incontinence articles are well-known in the art. Typically all these articles comprise a wearer facing surface and a garment facing surface. The wearer facing surface receives from the wearer of such articles liquids, bodily discharges such as menses, to be absorbed. In order for the article to store the liquid the wearer facing surface has to be liquid permeable while maintaining integrity of the outer wearer facing surface of the absorbent article. This wearer facing surface is provided by a topsheet and is the one which comes into contact with the wearer's skin.

Well-known topsheets in the art of absorbent articles are non-woven fabrics, woven fabrics or films. Films have to be rendered permeable by aperturing. Fabrics or non-woven fabrics are made of fibers which by their nature provide non-linear apertures of varying and changing size depending on the selected direction for liquid transport through them. Films are often made of polymeric material and typically comprise apertures which have been engineered to provide certain characteristics. These apertures can vary in shape and size. The walls of the apertures define the amount of extention - if any-beyond the plane of the film thickness and the direction of such extensions. The film apertures also can be provided in the shape of a funnel.

A typical topsheet made of polyethylene film has been successfully used in sanitary articles and adult incontinence products as well as inserts and baby diapers. One problem remaining is the total amount of liquid capable of passing through such a topsheet under usual usage conditions due to the total amount of open area of all apertures and individual aperture size and shape in particular. Exceptionally large apertures increase the liquid passage rate but pose the problem of masking because liquids such as menses remain visible to the wearer, which is considered undesirable. Also large apertures promote a backflow of absorbed liquid, so called rewet, which is undesiderable. Small individual apertures on the other hand cannot provide the liquid passage characteristics required to let liquids of high surface tensions pass through; this can be a problem in an absolute sense for very small apertures or cause too low a rate of liquid flow.

It also has been found that the total amount of open area for a given aperture size and shape is approximately linearly related to the rate of liquid passage. Again, masking of the liquid which has passed through but also material strength and other appearance considerations are limiting the extent as to which the total open area in a film topsheet can be selected.

EP-A-545423 describes an absorbent article having a composite topsheet comprising an upper layer formed by a perforated three dimensional film, and a lower layer formed by a thin flat meshy sheet comprising fibres which define smaller openings and obtained from a polymeric film.

From the aforesaid it is clear that a balancing problem between masking, material strength, other appearance considerations and total open area as well as individual aperture size and shape exists in the state of the art. The present invention does not attempt to provide selection criteria for this balancing problem but to shift the balance for this problem in order to obtain improved absorbent articles in respect to their capability of fast liquid intake as well as rewet and masking of the liquid received while maintaining acceptable characteristics of material strength and other considerations for topsheets.

It is hence an objective of the present invention to provide an apertured topsheet for absorbent articles which has larger apertures than those commonly found acceptable in film apertured topsheets while effectively improving or at least not deteriorating the rewet and masking of absorbed liquids.

### Description of the invention

The present invention provides an absorbent article having all the benefits of a large aperture film topsheet without the masking and rewet problems of the prior art. In particular, the absorbent article comprises a laminate topsheet having a wearer facing surface and a garment facing surface. The topsheet comprises a first and a second layer which are preferably joined to each other. An absorbent article generally further comprises a backsheet and an absorbent structure alternatively called absorbent core placed between the topsheet and the backsheet.

### Absorbent structure

The absorbent structure can include the following components: (a) optionally a primary fluid distribution layer preferably together with a secondary optional fluid distribution layer; (b) a fluid storage layer; (c) optionally a fibrous ("dusting") layer underlying the storage layer; and (d) other optional components.

### (a) Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers and even when wet are useful as distribution layers.

### (b) Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", "hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralized, polymeric gelling material. This material forms a hydrogel upon contact with water. Such polymer materials can be prepared form polymerizable, unsaturated, acid-containing monomers which are well known in the art.

Suitable carriers include materials which are conventionally utilised in absorbent structures such as natural, modified of synthetic fibers, particularly modified of non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins/panty liners.

An embodiment of the absorbent structure made according to the present invention comprises a double layer tissue laminate formed by folding the tissue onto itself. These layers can be joined to each other for example by adhesive or by mechanical interlocking or by hydrogel bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic of are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogeneous, i.e. have a distribution gradient in one or several directions within the dimensions of the storage layer. Non-homogeneous distribution can also refer to laminates of carriers enclosing absorbent gelling materials partially or fully.

### (c) Optional Fibrous ("Dusting") Layer

An optional component for inclusion in the absorbent structure according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent structure. Indeed, in those instances where the absorbent gelling material is in the form of macro-structures such as fibers, sheets or strips, this fibrous "dusting" layer need not be included. However, this "dusting" layer provides some additional fluid-handling capabilities such as rapid wicking of fluid along the length of the pad.

### (d) Other Optional Components of the absorbent structure

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent structure according to the invention and preferably is provided close to or as part of the primary or secondary fluid distribution layer are odor control agents. Active carbon coated with or in addition to other odor control agents, in particular suitable zeolite or clay materials, are optionally incorporated in the absorbent structure. These components can be incorporated in any desired form but often are included as discrete particles.

### Backsheet

The backsheet primarily prevents the exudates absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated non-woven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet can permit vapours to escape from the absorbent structure, i.e. be breathable, while still preventing exudates from passing through the backsheet. Also breathable backsheets comprising several layers, e.g. film plus non-woven structures, can be used.

### The topsheet

The term "joined", a used herein, encompasses configurations in which the first layer is directly secured to the second layer by affixing the first layer directly to the second layer; configurations in which the first layer is indirectly secured to the second layer by affixing the first layer to intermediate layer(s) which in turn is(are) affixed to the second layer. Both layers are preferably joined to each other across at least 25% of their total surface.

The layers of the topsheet can be joined together by adhesives, stitching, heat and/or pressure bonds, dynamic mechanical bonds, ultrasonic bonds, intermingling or entanglement of structural elements comprising the layers of the topsheet, such as by extruding one layer onto another, or by any other means known in the art.

The topsheet comprises a first passage layer which provides the user facing surface of the topsheet and a second passage layer between the first passage layer and the absorbent structure.

The topsheet as a whole and hence each layer individually needs to be compliant, soft feeling, and non-irritating to the wearer's skin. It also can have elastic characteristics allowing it to be stretched in one or two directions.

The first passage layer is provided by a film material having apertures which are referred herein as "large apertures" and optionally apertures which are referred herein as "small apertures". These apertures are provided to facilitate liquid transport from the wearer facing surface towards the absorbent structure.

For all measurements regarding the apertures in the first passage layer the plane of the smallest cross sectional areas of the aperture should be used, unless otherwise mentioned.

The large apertures have an individual open area of from 1.4 mm² to 3.0 mm² and preferably from 1.5 mm² to 2.5 mm². The total area of the large apertures in the upper passage layer excluding all other liquid transport apertures should be in the range from 5% to 20%, preferably from 10% to 20% of the surface area of the first layer of the topsheet.

The optional small apertures in the first layer of the topsheet of the absorbent article have an individual total open area of less than 1.4 mm² and typically not smaller than 0.15 mm². Apertures which are even smaller are usually not suitable for liquid transport at all and would only function as gas permeable apertures for example for breathability purposes. Preferably, the optional small apertures are in the range of 0.25 mm² to 0.4 mm².

The apertures are preferably substantially circular or polygonal. Their shape is limited by having a ratio of the largest to the smallest inner diagonal length in the range between 1 and 6, preferably 1 and 3. The total open area of all liquid transport apertures in the first layer is in the range of 10% to 40%, preferably 15% to 35% of the total area of the first layer. When approaching the upper limit of this total open area of the first layer, i.e. above a total open area of about 30%, the distribution of apertures needs to be homogeneous, i.e. there should not be a specific area where more aperture areas are concentrated than elsewhere. First passage layers not having a homogeneous distribution of liquid passage ways are also contemplated by the present invention and would habe a highest concentration of apertures in the area where liquid is expected to be discharged to.

The liquid transport apertures are formed in the film such that the walls to the apertures extend beyond the plane of the surface of the basic film, i.e. the film surface, before the film is apertured. The direction of these extending walls in the absorbent article is towards the garment facing surface of the article. The amount of extension of the walls of the apertures in the first layer is at least 0.3 mm beyond the film surface from which the walls of the apertures depend. Preferably the walls of the apertures form funnels of Venturi channels as is well-known in the art.

To ensure material stability the smallest distance between neighbouring apertures regardless of their particular shape and size is preferably at least 1.5 mm, preferably 2.0 mm. This distance is measured on the surface of the film on the side closest to the user facing surface of the absorbent article.

Also, as is typical for topsheets, the film material is preferably rendered hydrophilic to such a degree that the contact angle is less than 90° with distilled water upon first contact with the water. For films this can be achieved by surfactant treatment. For surfactant treated polymeric films providing the first layer it has been found that it is beneficial to use films where the surfactant is permanently fixed on the film surface. These are so-called film materials with resin integrated surfactant. For these films even repeated wetting by distilled water would provide approximately the same contact angle as the first contact with distilled water.

In another preferred execution of the first layer of the topsheet the wearer facing surface is treated with an agent such that liquids are directed towards the apertures. Such agents can be silicone or teflon which provide the treated surface with a self-cleaning effect. This treatment can be in addition to the above-mentioned surfactant treatment.

Films such as those disclosed in EP-0 205 286, EP-0 165 208, EP-0 18 020, EP-0 59 506 or US-3,929,135 are explicity referred to as suitable as first passage layer of the topsheet provided the requirements of the claims are met. Other suitable formed films, provided the requirements for the first passage layer are met, are described in U.S. Patent 4,324,246, U.S. Patent 4,342,314, U.S. Patent 4,463,045 and U.S. Patent 5,006,394. Particularly preferred microaperturing of formed film is disclosed in U.S. patent 4,609,518 and U.S. patent 4,629,643. These microapertures can also be included in the first passage layer of the topsheet provided they are less than 0.15 mm² and hence essentially provide breathability. Ways of making such films are well-known in the art and have also been disclosed in the above prior art references.

The second passage layer of the topsheet is provided by a second film material having apertures. Essentially this second passage layer can be identical to the first passage layer film except that large apertures should preferably be avoided or if some large apertures are present they should only be aligned with the large apertures in the first layer for up to 10% total open area, otherwise that could cause rewet and masking issues.

It is preferred that the second film material is at least as hydrophilic as the film material of the first layer in order to present no barrier for the liquid. Better yet it is more hydrophilic (or les hydrophobic) than the first film and creates a directing force for the liquid towards the absorbent structure after passing the first layer.

It is also important that both layers have about the same total open area. Preferably the second layer has 10% larger, most preferably a more than 20% larger total oper area than the first film layer.

Since both layers of the topsheet, individually, are well-known in the art it is not necessary to elaborate on further details of the topsheet layers of the present invention but exemplify the performance of the here-to-forth unknown dual layer topsheet combination in the following examples:

The tests to analyse liquid intake rate, rewet and masking are simple tests to review the specific performance aspect of a product in relation to an alternative product. The results are intended to identify the qualitative difference between alternative executions and give an indication of the relative magnitude of improvements. Since the topsheet combination distinguishes the present invention from the prior art a backsheet and core combination commercially available with usual characteristics will typically be sufficient to evaluate alternative topsheet combinations.

For the tests described below a commercially available sanitary napkin having a polyethylene backsheet and a tissue laminate (tissue folded around and onto itself including an absorbent gel material) was used as a base in combination with alternative topsheet combinations.

The tested topsheet was made of a first film layer glued by a hotmelt adhesive to a fibrous layer, the second layer according to the present invention. The hotmelt adhesive was applied in a quantity of 3 g/m² by spiral gluing and the adhesive which was used was H2031, by Findley Euro B.V., from Netherlands.

The reference film layer was "DriWeave" film available from The Procter & Gamble Company, Cincinnati, Ohio, USA and commercialised on their Always (R) and Aldays (R) brands. This film is essentially identical with the first layer of the topsheet of the present invention but for the aperture size. Apertures in the reference film would only qualify as small apertures since they had 0.34 mm² open area. The total open area was 31.28% with 92 apertures per cm².

The first layer film according to the present invention had circular, slightly funnel shaped apertures of 1.7 mm² area for the large apertures and 0.20 mm² for the small apertures. The total open area was 32.82% with 11.24% due to the large apertures and 21.58% due to the small apertures. The average number of apertures per cm² was 6.61 for the large apertures and 107.9 for the small apertures. The walls of the apertures including the basic film has a caliper of 0.427 mm while the film thickness was 0.0255 mm (such that the wall extended about 0.4 mm from the plane of the film).

The second layer according to the present invention was the above described "Dri Weave" film.

The reference second layer is available from Suominen under reference F3200. It provides a caliper of 0.5 mm and basis weight of 50 g/m² at a void volume of 25%.

The following test samples were prepared in sufficient quantity for statistical analysis of the test results:

| Product Code: | A | B |
|---|---|---|
| first layer of topsheet | reference | large aperture film according to present invention |
| second layer of topsheet | reference | Dri Weave |
| Core + Backsheet | - commercially available core plus backsheet of Always Ultra Medium Size sanitary napkins - | |

The tests were an acquisition test and visual inspection on all test samples as to masking.

### Acquisition test procedure

This procedure measures a product's ability to "keep on absorbing" (acquisition decay) subject to a repeat assaults of fluid under a prescribed set of conditions. This procedure is recommended for multiple product comparisons.

This method evaluates the time required for the acquisition of given amounts of liquid during repeated imbibitions(three in this case), at relatively high speed (about 3 ml/sec) and under pressure of 1723.7 Pa (0.25 PSI), to model "in use" pressure while wearing.

Each product is laid down on a flat surface and an acquisition plate is placed on it. The acquisition plate comprises a rectangular plexiglass plate 70 x 220 x 8 mm with an aperture 22 mm in diameter formed therein. A cylinder 45 mm high and 22 mm in internal diameter is located over the aperture in sealing contact with the plate. The cylinder is filled with Artificial Menstrual Fluid (AMF), and a pressure of 1723.7 Pa (0.25 PSI) is applied to the plate, obtained with appropriate weights positioned on the plate, the pressure being that measured with reference to the portion of the product under the acquisition plate. The acquisition time is the time from the beginning of each imbibition until the disappearance of the liquid from the interior of the cylinder. A waiting time of 10 minutes is left after each imbibition before repeating the procedure.

| Rewetting test procedure |
|---|
| Drop 7 ml of Artificial Menstrual Fluid (AMF) over 90 secs onto the centre of a sanitary napkin. |
| Dropping area is 3x4 sqcm, with 4 cm being in the longitudinal direction of the sanitary napkin. |
| Let the pad stand for 20 min. |
| Place 7 layers of proweighed blotting paper (*) in the centre of the pad. |
| Apply the pressure of 70 g/sqcm. |
| Wait for 15 secs with the pressure applied. |
| Remove the blotting paper and measure the weight. |
| Final rewet result = Sum up the weight of liquid on 7 blotting papers. |

| |
|---|
| (*) blotting paper: from Schleicher & Schuell, code 597 Ref. No. 311812 - DASSEL - GERMANY |

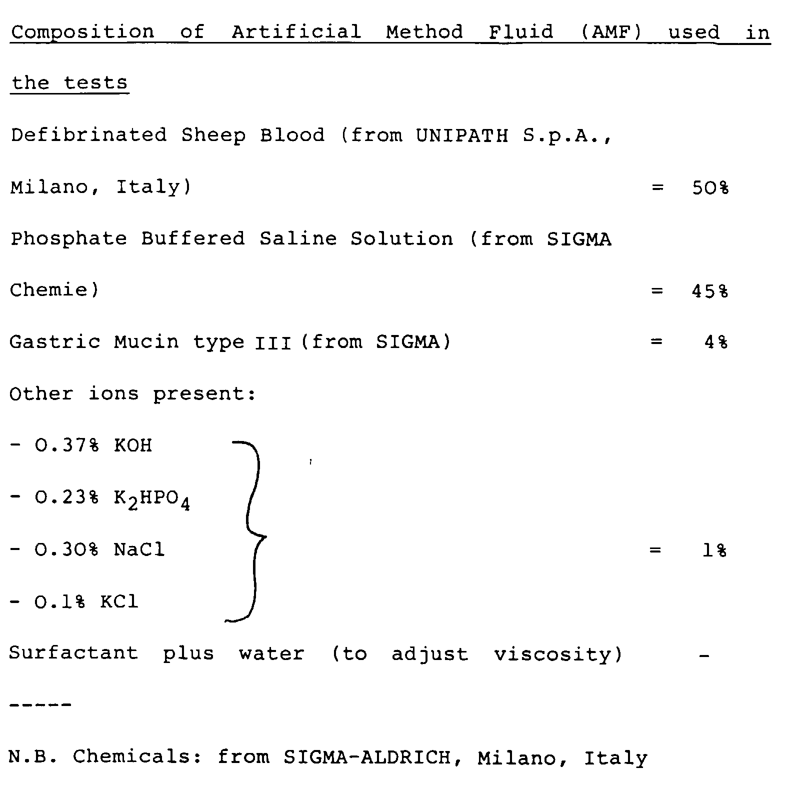

### Test results

The test results for acquisition and rewet can be found as index % in the following tables.

From visual inspection no deterioration of the masking was detectable between product B and product A. It is in fact better.

| Acquisition time results (index %) | | | |
|---|---|---|---|
| Testfluid at viscosity of 7m Pas Cumulative amount of Liquid | 5 ml | 10 ml | 15 ml |
| Product A | 180% | 200% | 250% |
| Product B-basis | 100% | 100% | 100% |

### Rewet

In respect to rewet the problem prejudically expected with large aperture film topsheets is shown by the following rewet data. The topsheet according to the present invention addresses this problem and provides even an improvement over the small aperture topsheet reference.

| Rewet index test fluid at viscosity of 11 m Pas | |
|---|---|
| Product A | 360% |
| Product B-basis | 100% |

This result shows that the combination of the layers according to the present invention can provide the desired benefit of acquisition speed while a prior art combination fails.

## Claims

1. Absorbent article, having fast liquid intake, low rewet, and good masking performance, said article comprising a topsheet, a backsheet, and an absorbent structure placed between said topsheet and said backsheet, said topsheet having a wearer facing surface and a garment facing surface and said topsheet comprising
- a first passage layer, said first passage layer providing said user facing surface of said topsheet, and
- a second passage layer, said second passage layer being placed between said first passage layer and said absorbent structure,
- both said passage layers being preferably joined to each other,
- said first passage layer being provided by a first film material having large apertures for liquid transport,
- said large apertures having an individual open area in the range of from 1.4 mm² to 3.0 mm²,
- said large apertures having a total open area in the range of from 5% to 20% of the total area of said first passage layer,
- said liquid transport apertures having a largest inner diagonal length and a smallest inner diagonal length, the ratio of said largest to said smallest inner diagonal length being in the range of from 1 to 6,
- said liquid transport apertures having inner walls which depend at least 0.3 mm from the surface of said first film material, said inner walls depending in a direction towards said garment facing surface,
- said first film material being rendered hydrophilic such that it forms a contact angle of less than 90 degrees with distilled water upon first contact with distilled water,
- the second passage layer being provided by a second film material comprising small apertures for liquid transport,
- said small apertures in said second film material having an individual area in the range of from 0.15 mm² to less than 1.4 mm²,
- said small apertures in said second film material having a total open area in the range of from 10% to 40% of the total area of said second passage layer,
- said small apertures in said second film materials having a largest inner diagonal length and a smallest inner diagonal length, the ratio of said largest to said smallest inner diagonal length being in the range of from 1 to 6,
- said second film material being preferably at least as hydrophilic as said first film material.
said absorbent article characterized in that:
- said small apertures in said second film material have inner walls which depend at least 0.3 mm from the surface of said second film, said inner walls preferably depending in a direction towards said absorbent structure.

2. Absorbent article according to claim 1 wherein said second film material has only small apertures.

3. Absorbent article according to any of the preceding claims, wherein said large apertures have an individual open area in the range from 1.5 mm² to 2.5 mm².

4. Absorbent article according to any of the preceding claims wherein said large apertures have a total open area in the range from 10% to 20% of the total area of said first passage layer.

5. Absorbent article according to any of the preceding claims wherein said first passage layer is provided by a film material further having small apertures for liquid transport,
- said small apertures in said first passage layer having an individual open area in the range of from 0.15 mm² to less than 1.4 mm²,
- the total open area of all said large and said small liquid transport apertures in said first passage layer being in the range of from 10% to 40% of the total area of said first passage layer.

6. Absorbent article according to claim 5 wherein said small apertures in said first passage layer having an individual area in the range of from 0.25 mm² to 0.4 mm².

7. Absorbent article according to claim 5 or 6 wherein the total open area of all said large and said small liquid transport apertures in said first passage layer is in the range of from 15% to 35% of the total area of said first passage layer.

8. Absorbent article according to any of the preceding claims wherein the smallest edge to edge distance between apertures in said first passage layer is at least 1.5 mm.

9. Absorbent article according to claim 8 wherein the smallest edge to edge distance between apertures in said first passage layer is at least 2.0 mm.

10. Absorbent article according to any of the preceding claims wherein said small apertures in said second passage layer have an individual open area in the range of from 0.25 mm² to 0.4 mm².

11. Absorbent article according to any of the preceding claims wherein the total open area of all said small apertures in said second passsage layer is in the range of from 15% to 35% of the total area of said second passage layer.

12. Absorbent article according to any of the preceding claims wherein the smallest edge to edge distance between apertures in said second passage layer is at least 1.5 mm.

13. Absorbent article according to claim 12 wherein the smallest edge to edge distance between apertures in said second passage layer is at least 2.0 mm.

14. Absorbent article according to any of the preceding claims wherein both said passage layers are joined directly to each other.

## Patentansprüche

1. Absorbierender Artikel mit schneller Flüssigkeitsaufnahme, geringer Rücknässung und gutem Abdeckvermögen, wobei der Artikel eine Oberschicht, eine Unterschicht und eine zwischen der Oberschicht und der Unterschicht angeordnete absorbierende Struktur umfaßt, wobei die Oberschicht eine trägerseitige Oberfläche und eine wäscheseitige Oberfläche aufweist und die Oberschicht umfaßt
- eine erste Durchgangslage, wobei die erste Durchgangsschicht die benutzerseitige Oberfläche der Oberschicht liefert, und
- eine zweite Durchgangslage, wobei die zweite Durchgangsschicht zwischen der ersten Durchgangslage und der absorbierenden Struktur angeordnet ist,
- beide Durchgangslagen vorzugsweise miteinander verbunden sind,
- die erste Durchgangslage durch ein erstes Filmmaterial mit großen Öffnungen für den Flüssigkeitstransport geliefert wird,
- wobei die großen Öffnungen eine individuelle Öffnungsfläche im Bereich von 1,4 mm² bis 3,0 mm² haben,
- wobei die großen Öffnungen eine Gesamtöffnungsfläche im Bereich von 5% bis 20% der Gesamtfläche der ersten Durchgangslage haben,
- wobei die Flüssigkeits-Transportöffnungen eine größte innere diagonale Länge und eine kleinste innere diagonale Länge haben, wobei das Verhältnis der größten zu der kleinsten inneren diagonalen Länge im Bereich von 1 bis 6 liegt,
- wobei die Flüssigkeits-Transportöffnungen Innenwände haben, welche um wenigstens 0,3 mm von der Oberfläche des ersten Filmmaterials abhängen, wobei die Innenwände in einer Richtung zur wäscheseitigen Oberfläche abhängen,
- wobei das erste Filmmaterial derart hydrophil gemacht ist, daß es mit destilliertem Wasser einen Berührungswinkel von weniger als 90 Grad beim ersten Kontakt mit dem destillierten Wasser bildet,
- wobei die zweite Durchgangslage durch ein zweites Filmmaterial geliefert wird, das kleine Öffnungen für den Flüssigkeitstransport umfaßt,
- wobei die kleinen Öffnungen in dem zweiten Filmmaterial eine individuelle Fläche im Bereich von 0,15 mm² bis weniger als 1,4 mm² haben,
- wobei die kleinen Öffnungen in dem zweiten Filmmaterial eine Gesamtöffnungsfläche im Bereich von 10% bis 40% der Gesamtfläche der zweiten Durchgangslage haben,
- wobei die kleinen Öffnungen im zweiten Filmmaterial eine größte innere diagonale Länge und eine kleinste innere diagonale Länge haben, wobei das Verhältnis der größten zur kleinsten inneren diagonalen Länge im Bereich von 1 bis 6 liegt,
- wobei das zweite Filmmaterial vorzugsweise wenigstens so hydrophil ist, wie das erste Filmmaterial,
- wobei der absorbierende Artikel dadurch gekennzeichnet ist, daß:
- die kleinen Öffnungen in dem zweiten Filmmaterial Innenwände haben, welche um wenigstens 0,3 mm von der Oberfläche des zweiten Films herabhängen, wobei die Innenwände vorzugsweise in einer Richtung zu der absorbierenden Struktur herabhängen.

2. Absorbierender Artikel nach Anspruch 1, in welchem das zweite Filmmaterial nur kleine Öffnungen hat.

3. Absorbierender Artikel nach einem der vorstehenden Ansprüche, in welchem die großen Öffnungen eine individuelle Öffnungsfläche im Bereich von 1,5 mm² bis 2,5 mm² haben.

4. Absorbierender Artikel nach einem der vorstehenden Ansprüche, in welchem die großen Öffnungen eine Gesamtöffnungsfläche im Bereich von 10% bis 20% der Gesamtfläche der ersten Durchgangslage haben.

5. Absorbierender Artikel nach einem der vorstehenden Ansprüche, in welchem die erste Durchgangslage durch ein Filmmaterial geliefert wird, das ferner kleine Öffnungen zum Flüssigkeitstransport hat,
- wobei die kleinen Öffnungen in der ersten Durchgangslage eine individuelle Öffnungsfläche im Bereich von 0,15 mm² bis weniger als 1,4 mm² haben,
- wobei die Gesamtöffnungsfläche aller großen und kleinen Flüssigkeits-Transportöffnungen in der ersten Durchgangslage im Bereich von 10% bis 40% der Gesamtfläche der ersten Durchgangslage liegt.

6. Absorbierender Artikel nach Anspruch 5, in welchem die kleinen Öffnungen in der ersten Durchgangslage eine individuelle Fläche im Bereich von 0,25 mm² bis 0,4 mm² haben.

7. Absorbierender Artikel nach Anspruch 5 oder 6, in welchem die Gesamtöffnungsfläche aller großen und kleinen Flüssigkeits-Transportöffnungen in der ersten Durchgangslage im Bereich von 5% bis 36% der Gesamtfläche der ersten Durchgangslage liegt.

8. Absorbierender Artikel nach einem der vorstehenden Ansprüche, in welchem die kleinste Rand-zu-Rand-Entfernung zwischen Öffnungen in der ersten Durchgangslage wenigstens 1,5 mm beträgt.

9. Absorbierender Artikel nach Anspruch 8, in welchem die kleinste Rand-zu-Rand-Entfernung zwischen Öffnungen in der ersten Durchgangslage wenigstens 2,0 mm beträgt.

10. Absorbierender Artikel nach einem der vorstehenden Ansprüche, in welchem die kleinsten Öffnungen in der zweiten Durchgangslage eine individuelle Öffnungsfläche im Bereich von 0,25 mm² bis 0,4 mm² haben.

11. Absorbierender Artikel nach einem der vorstehenden Ansprüche, in welchem die Gesamtöffnungsfläche aller kleinen Öffnungen in der zweiten Durchgangslage im Bereich von 15% bis 35% der Gesamtfläche der zweiten Durchgangslage liegt.

12. Absorbierender Artikel nach einem der vorstehenden Ansprüche, in welchem die kleinste Rand-zu-Rand-Entfernung zwischen Öffnungen in der zweiten Durchgangslage wenigstens 1,5 mm beträgt.

13. Absorbierender Artikel nach Anspruch 12, in welchem die kleinste Rand-zu-Rand-Entfernung zwischen Öffnungen in der zweiten Durchgangslage wenigstens 2,0 mm beträgt.

14. Absorbierender Artikel nach einem der vorstehenden Ansprüche, in welchem beide Durchgangslagen direkt miteinander verbunden sind.

## Revendications

1. Article absorbant ayant une admission de liquide rapide, un remouillage faible et une bonne performance de masquage, ledit article comprenant une feuille de dessus, une feuille de fond et une structure absorbante placée entre ladite feuille de dessus et ladite feuille de fond, ladite feuille de dessus ayant une surface faisant face au porteur et une surface faisant face au vêtement, et ladite feuille de dessus comprenant :
- une première couche de passage, ladite première couche de passage fournissant ladite surface faisant face à l'utilisateur de ladite feuille de dessus, et
- une deuxième couche de passage, ladite deuxième couche de passage étant placée entre ladite première couche de passage et ladite structure absorbante,
- les deux dites couches de passage étant réunies, de préférence, l'une à l'autre,
- ladite première couche de passage étant constituée d'un premier matériau de film ayant de grands orifices pour le transport de liquide,
- lesdits grands orifices ayant une surface ouverte individuelle comprise dans la plage allant de 1,4 mm² à 3,0 mm²,
- lesdits grands orifices ayant une surface ouverte totale comprise dans la plage allant de 5 % à 20 % de la surface totale de ladite première couche de passage,
- lesdits orifices de transport de liquide ayant une longueur diagonale intérieure la plus grande et une longueur diagonale intérieure la plus petite, le rapport entre lesdites longueurs diagonales intérieures la plus grande et la plus petite étant compris dans la plage allant de 1 à 6,
- lesdits orifices de transport de liquide ayant des parois intérieures qui pendent au moins de 0,3 mm de la surface dudit premier matériau de film, lesdites parois intérieures pendant dans une direction orientée vers ladite surface faisant face au vêtement,
- ledit premier matériau de film étant rendu hydrophile de façon qu'il forme un angle de contact inférieur à 90 degrés avec de l'eau distillée lors d'un premier contact avec de l'eau distillée,
- la deuxième couche de passage étant constituée d'un deuxième matériau de film comprenant de petits orifices pour le transport de liquide,
- lesdits petits orifices dudit deuxième matériau de film ayant une surface individuelle comprise dans la plage allant de 0,15 mm² à moins de 1,4 mm²,
- lesdits petits orifices dudit deuxième matériau de film ayant une surface ouverte totale comprise dans la plage allant de 10 % à 40 % de la surface totale de ladite deuxième couche de passage,
- lesdits petits orifices dudit deuxième matériau de film ayant une longueur diagonale intérieure la plus grande et une longueur diagonale intérieure la plus petite, le rapport entre lesdites longueurs diagonales intérieures la plus grande et la plus petite étant compris dans la plage allant de 1 à 6,
- ledit deuxième matériau de film étant, de préférence, au moins aussi hydrophile que ledit premier matériau de film,
ledit article absorbant étant caractérisé en ce que :
- lesdits petits orifices dudit deuxième matériau de film ont des parois intérieures qui pendent au moins de 0,3 mm de la surface dudit deuxième film, lesdites parois intérieures pendant, de préférence, dans une direction orientée vers ladite structure absorbante.

2. Article absorbant selon la revendication 1, dans lequel ledit deuxième matériau de film ne présente que des petits orifices.

3. Article absorbant selon l'une quelconque des revendications précédentes, dans lequel lesdits grands orifices ont une surface ouverte individuelle comprise dans la plage allant de 1,5 mm² à 2,5 mm².

4. Article absorbant selon l'une quelconque des revendications précédentes, dans lequel lesdits grands orifices ont une surface ouverte totale comprise dans la plage allant de 10 % à 20 % de la surface totale de ladite première couche de passage.

5. Article absorbant selon l'une quelconque des revendications précédentes, dans lequel ladite première couche de passage est constituée d'un matériau de film ayant, en outre, de petits orifices pour le transport de liquide,
- lesdits petits orifices de ladite première couche de passage ayant une surface ouverte individuelle comprise dans la plage allant de 0,15 mm² à moins de 1,4 mm²,
- la surface ouverte totale de tous lesdits grands et lesdits petits orifices de transport de liquide de ladite première couche de passage étant comprise dans la plage allant de 10 % à 40 % de la surface totale de ladite première couche de passage.

6. Article absorbant selon la revendication 5, dans lequel lesdits petits orifices de ladite première couche de passage ont une surface individuelle comprise dans la plage allant de 0,25 mm² à 0,4 mm².

7. Article absorbant selon la revendication 5 ou 6, dans lequel la surface ouverte totale de tous lesdits grands et lesdits petits orifices de transport de liquide de ladite première couche de passage est comprise dans la plage allant de 15 % à 35 % de la surface totale de ladite première couche de passage.

8. Article absorbant selon l'une quelconque des revendications précédentes, dans lequel la plus petite distance bord à bord entre les orifices de ladite première couche de passage est au moins de 1,5 mm.

9. Article absorbant selon la revendication 8, dans lequel la plus petite distance bord à bord entre les orifices de ladite première couche de passage est au moins de 2,0 mm.

10. Article absorbant selon l'une quelconque des revendications précédentes, dans lequel lesdits petits orifices de ladite deuxième couche de passage ont une surface ouverte individuelle comprise dans la plage allant de 0,25 mm² à 0,4 mm².

11. Article absorbant selon l'une quelconque des revendications précédentes, dans lequel la surface ouverte totale de tous lesdits petits orifices de ladite deuxième couche de passage est comprise dans la plage allant de 15 % à 35 % de la surface totale de ladite deuxième couche de passage.

12. Article absorbant selon l'une quelconque des revendications précédentes, dans lequel la plus petite distance bord à bord entre les orifices de ladite deuxième couche de passage est au moins de 1,5 mm.

13. Article absorbant selon la revendication 12, dans lequel la plus petite distance bord à bord entre les orifices de ladite deuxième couche de passage est au moins de 2,0 mm.

14. Article absorbant selon l'une quelconque des revendications précédentes, dans lequel les deux couches de passage sont réunies directement l'une à l'autre.
